# EUROPEAN PATENT APPLICATION

(11) **EP 3 616 744 A1**
(43) Date of publication of application: **04.03.2020**
(21) Application number: 18791681.2
(22) Date of filing: 30.03.2018
(51) Int. Cl.: A61M 37/00

(54) **MICRONEEDLE PRODUCTION METHOD**

(30) Priority: 27.04.2017 JP 2017088370
(71) Applicant: Think-Lands Co., Ltd., Yokohama-city, Kanagawa 230-0046 (JP)
(72) Inventor: MIYAJI Kunio, Yokohama-city Kanagawa 230-0046 (JP); OIKAWA Yoichi, Yokohama-city Kanagawa 230-0046 (JP); TOYODA Kohei, Yokohama-city Kanagawa 230-0046 (JP)
(74) Representative: Plougmann Vingtoft a/s
(86) International application number: PCT/JP2018/013740
(87) International publication number: WO 2018/198675

(57) **Abstract**

[Problem] It is possible to produce hollow microneedles using workpiece containing macromolecules as a major component.

[Solution] A method of producing microneedles in the present invention includes a molding step of passing a thin pillar array member through through-holes to be used for positioning, and determining the shape of microneedles in the state where the pillar array member penetrates a liquid macromolecular compound in a liquid and semi-liquid state, a solidification step of solidifying the macromolecular compound, and a pull-out step of pulling out the pillar array member.

## Description

### Technical Field

The present invention is preferably applied to a method of producing hollow microneedles using bioabsorbable polymer materials, for example.

### Background Art

Microneedles produced using macromolecular compounds such as bioabsorbable polymers have been widely known for cosmetic purposes or others (for example, see PTL1).

### Citation List

### Patent Literature

PTL1: Japanese Patent No. 5495034

### Summary of Invention

### Technical Problem

The aforementioned microneedles, however, are directly made of target substances. Since not all materials are usable as the target substances that are formed into microneedles, there has been a demand for hollow microneedles produced using a macromolecular compound.

The present invention has been made to solve the above problem, and intends to provide a method of producing hollow microneedles using a macromolecular compound.

### Solution to Problem

To solve the above problem, a microneedle production method according to the present invention includes: a molding step of passing a thin pillar array member through through-holes, and determining a shape of microneedles in the state where the pillar array member penetrates a liquid macromolecular compound in a liquid or semi-liquid state; a solidification step of solidifying the macromolecular compound; and a pull-out step of pulling out the pillar array member.

### Advantageous Effects of Invention

The present invention provides a method of producing hollow microneedles using a macromolecular compound.

### Brief Description of Drawings

[FIG. 1] FIG. 1 provides schematic views of a conventional method of producing microneedles.
[FIG. 2] FIG. 2 provides schematic views illustrating structures of a mold and a pillar array member according to a first embodiment.
[FIG. 3] FIG. 3 is a schematic view illustrating the pillar array member passing through through-holes according to the first embodiment.
[FIG. 4] FIG. 4 provides schematic views for explaining how to pull out the pillar array member according to the first embodiment.
[FIG. 5] FIG. 5 is a schematic view illustrating a structure of a microneedle sheet according to the first embodiment.
[FIG. 6] FIG. 6 is a schematic view illustrating passing (1) of a pillar array member through through-holes according to a second embodiment.
[FIG. 7] FIG. 7 is a schematic view illustrating molding (1) of workpiece according to the second embodiment.
[FIG. 8] FIG. 8 is a schematic view illustrating the molding (2) of the workpiece according to the second embodiment.
[FIG. 9] FIG. 9 is a schematic view illustrating a structure of a microneedle sheet according to the second embodiment.
[FIG. 10] FIG. 10 is a flowchart for explaining a method of producing microneedles.

### Description of Embodiments

### (First Embodiment)

Embodiments of the present invention will now be described with reference to the accompanying drawings.

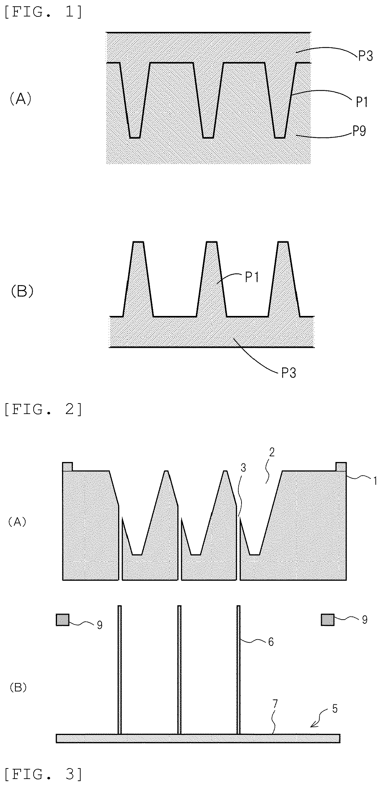

For cosmetic or medical uses, microneedles have been proposed over recent years, which consist of micro-projections with small diameter, aiming to deliver cosmetic ingredients or medical drugs as target substances into skin with as little injury to the skin as possible (for example, please see Japanese Patent No. 5495034). As illustrated in FIG. 1, a microneedle P1 is produced by pattern transfer using a mold P9 or the like. In general, a plurality of microneedles P1 are produced on a base sheet P3.

These microneedles themselves contain target substances, and are absorbed into body as they are when inserted into skin. Note now that microneedles are limited in the kind and amount of target substances to be contained therein.

The inventors of the present application have found a method of producing microneedles that each have an opening formed therein to allow target substances to be injected into body through the opening.

As illustrated in FIG. 2, a method of producing microneedles in the present invention uses a mold 1 having a transfer pattern for microneedles, a pillar array member 5, and spacers 9. The mold 1 has projection-shaped recesses 2 that form the transfer pattern for microneedles, and through-holes 3 passing through the projection-shaped recesses 2 to the bottom surface (opposite the projection-shaped recesses 2) of the mold 1. One through-hole 3 is formed in every projection-shaped recess 2. The material for the mold 1 is not limited to any particular material and publicly-known various materials, including metal, wood, and resin materials, are usable.

The pillar array member 5 includes a plurality of pillars 6 placed at positions matching those of the through-holes 3, and a planar plate 7. The pillars 6 are fixed to the plate 7 so as to stick out therefrom. The materials for the plate 7 and pillars 6 are not limited to any particular materials, and publicly-known various materials, including metal, wood, and resin materials, are usable. For example, the pillars 6 are fixed to the plate 7 with hooks, such as screws.

The spacers 9 are designed to be sandwiched between the pillar array member 5 and the mold 1, in order to form a prescribed gap space between the pillar array member 5 and the mold 1. Note that the spacers 9 are freely removable from between the pillar array member 5 and the mold 1.

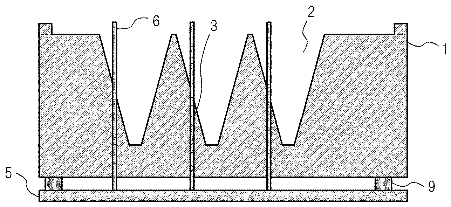

As illustrated in FIG. 3, first, the pillar array member 5 is inserted into the mold 1 and the spacers 9 are placed between the pillar array member 5 and the mold 1. By doing so, the pillar array member 5 passes through the through-holes 3 of the mold 1 and projects from the mold 1, with leaving a prescribed gap space between the pillar array member 5 and the mold 1.

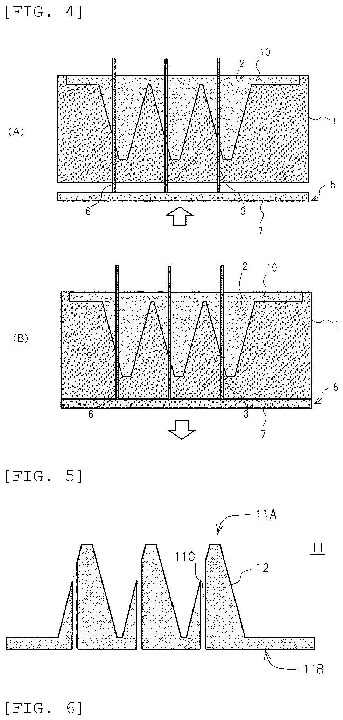

As illustrated in FIG. 4, the mold 1 having the pillar array member 5 inserted therein is filled with workpiece 10. At this time, the workpiece 10 is in a liquid or semi-liquid state. In this connection, the liquid or semi-liquid state is a state where the workpiece has a viscosity of 10 to 30000 mPa·s at 60 rpm at a temperature enabling the filling. Such states range from a liquid state with high liquidity to a gel state with high thixotropy. Then, the workpiece 10 is solidified in the state where the pillar array member 5 projects from the workpiece 10. Any method may be employed for the solidification. For example, the workpiece 10 may be solidified by cooling the workpiece 10 that has been liquefied by heating, by causing a low-molecular workpiece 10 to undergo chemical reaction (polymerization) with a crosslinking agent or curing agent, by crystallization, by evaporation of a solvent component, or by another method.

The workpiece 10 in solidified state contains a macromolecular compound as a major component. The macromolecular compound described herein refers to an organic compound having an average molecular weight Mw of 5000 or more. The major component takes a role of a chemical bone structure for the workpiece and means that the macromolecular compound is preferably contained by 50 weight percent or more in the whole workpiece. In this connection, a solvent component whose average molecular weight Mw is less than 300, such as water or organic solvent, is not included in the total weight. For example, solvent components, like sodium hyaluronatem, collagen, and cellulose, which have a property of embracing specific molecules (solvent components), such as water molecules, are excluded from the total weight. The macromolecular compound preferably has a Tg (glass transition point) of 50°C or higher, and more preferably, 70°C or higher. This is because, when Tg is low, it is not possible to treat the macromolecular compound easily at ambient temperature.

As the macromolecular compound, only one type of macromolecules may be contained, or two or more types of macromolecules may be contained. Note that the ratio of the major component refers to a weight ratio in the workpiece obtained after all processing steps for the workpiece are completed, and so-called solvent components that are intentionally evaporated in a drying process after formation of micro-projections are not taken into account here. That is, the ratio of the major component is a ratio in the workpiece obtained after the processing for the workpiece is completed.

As the macromolecular compound, known compounds (synthetic polymer and naturally-occurring polymer) are usable. For example, bioabsorbable polymers, as well as various plastic materials, such as PET (polyethylene terephthalate), polyethylene, polypropylene, acrylic resin, epoxy resin, and polystyrene, are usable.

As the bioabsorbable polymer, known compounds (synthetic polymer and naturally-occurring polymer) are usable. Examples of the bioabsorbable polymer include ester compounds such as polylactic acid, polyglycolic acid, poly-s-caprolactone, poly-p-dioxane, and poly(malic acid), acid anhydride such as polyacid anhydride, orthoester compounds such as polyorthoester, carbonate compounds such as polycarbonate, phosphazene compounds such as poly(diaminophosphazene), peptide compounds such as synthetic polypeptide, phosphate ester compounds such as polyphosphoester urethane, carbon-carbon compounds such as polycyanoacrylate, poly-β-hydroxybutyric acid, ester compounds such as poly(malic acid), polyamino acids, chitin, chitosan, hyaluronic acid, sodium hyaluronate, pectic acid, galactan, starch, dextran, dextrin, alginic acid, sodium alginate, cellulose compounds (ethyl cellulose, carboxymethyl cellulose, hydroxy ethyl cellulose, hydroxypropyl cellulose, hydroxypropyl methyl cellulose, methyl cellulose), glycoside compounds (polysaccharides) such as gelatin, agar, Keltrol, Rheozan, xanthan gum, pullulan, and gum arabic, peptide compounds (peptide, protein) such as collagen, gelatin, fabrin, gluten, and serum albumin, phosphate ester compounds (nucleic acids) such as deoxyribonucleic acid and ribonucleic acid, and vinyl compounds such as polyvinyl alcohol.

After the solidification, the workpiece 10 is removed as a molded product from the mold 1. If the workpiece 10 is removed with the pillar array member 5 remaining inserted therein, the workpiece 10 may be deformed or the pillars 6 may be broken. In view of this, in the present invention, the pillar array member 5 is first pulled out and then the workpiece 10 is removed.

Note that the workpiece 10 in a liquid or semi-liquid state fills tiny concavities on the surfaces of the pillars 6, so that the workpiece 10 and the pillars 6 have high affinity to each other. Therefore, some force is needed to pull them apart. If the pillar array member 5 is pulled in a direction separated from the mold 1, the pillar array member 5 is powerfully pulled out with great force at first. If the workpiece 10 is hard, the pillar array member 5 would not be pulled out but would be broken in the middle. If the workpiece is soft, on the other hand, the tips of the openings would be blocked with part of the workpiece 10 attaching to the pillars 6.

To deal with this, as illustrated in (A) and (B) of FIG. 4, before pulling the pillar array member 5 out of the mold 1, the spacers 9 are first removed, and the pillar array member 5 is moved once toward the mold 1. Then, the pillar array member 5 is pulled out of the mold 1.

This causes the pillars 6 and the workpiece 10 to lose the affinity therebetween and pushes part of the workpiece 10 attaching to the pillar array member 5 toward the opposite side. After doing so, it is possible to pull the pillar array member 5 out of the mold 1 gently and slowly.

Then, the workpiece 10 is removed from the mold 1. As a result, a microneedle sheet 11 is produced as a molded product. This microneedle sheet 11 is expected so that the microneedles 12 are inserted into skin as injection needles for medical or cosmetic purposes.

In the case of using the microneedles as injection needles, the microneedle sheet 11 is cut into pieces of desired size according to necessity, and an applicator containing target substances is set at the bottom surface 11B and caps (not illustrated) that are easily dissolved when the microneedles are inserted into body are attached to the tips 11A of openings 11C so as not to let the target substances leak while the microneedles are not in use.

Since the mold 1 and the pillars 6 are configured to be easily removed from each other, as described above, it is possible to easily produce a hollow-microneedle sheet 11 using a mold method. In addition, it is not necessary to form projections in the mold 1, which reduces the production cost for the mold 1 and increases the durability of the mold 1. In addition, it is possible to give flexibility in the size and position of hollows.

### (Second Embodiment)

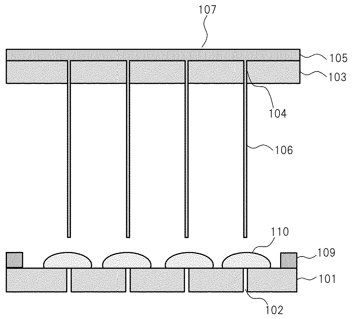

The following describes a second embodiment with reference to FIGS. 6 to 9. The second embodiment is different from the first embodiment described with reference to FIGS. 1 to 5 in a method of processing the workpiece. Parts in the second embodiment are given reference numerals obtained by adding 100 to the reference numerals of corresponding parts of the first embodiment, and the same parts as the first embodiment will not be explained again.

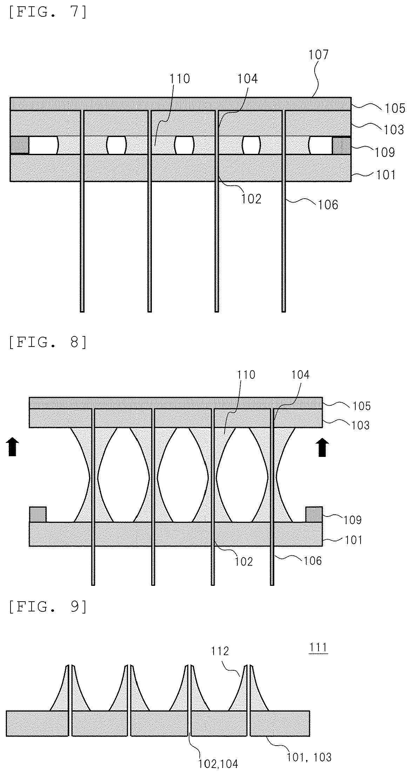

In this embodiment, through-holes 102 and 104 are formed in plate-shaped base sheets 101 and 103, as illustrated in FIG. 6. Each base sheet 101 and 103 serves as the sheet of a microneedle sheet 111 (refer to FIG. 9), which is a molded product, and any materials are usable for the base sheets 101 and 103. Various kinds of materials, such as metal and resin materials, are usable, but a material with good wettability and good adhesion on workpiece 110 is preferable. To improve the wettability and adhesion, the surfaces of the base sheets 101 and 103 may be modified, for example, by making the surfaces of the base sheets 101 and 103 bumpy, by performing plasma treatment, by applying an anchor agent, a primer agent, an adhesive agent, or the like. In this connection, if more rigidity is desired in the base sheet 101, a supporting plate with through-holes formed therein as in the base sheet 101 may be laminated on the base sheet 101.

The workpiece 110 in a liquid or semi-liquid state with prescribed viscosity is placed in the form of a particle on each through-hole 102 of the base sheet 101. In addition, spacers 109 are placed on the base sheet 101. In this connection, the amount of a particle of the workpiece 110 to be placed is approximately twice the volume of a microneedle. The liquid or semi-liquid state is a state where the workpiece has a viscosity of 300 to 30000 mPa·s at 6 rpm at ambient temperature (25°C), and more preferably 1000 to 20000 mPa·s. Such states range from a liquid state with high liquidity to a gel state with high thixotropy. Pillars 106 of a pillar array member 105 are passed through the respective through-holes 104 of the base sheet 103 in advance, and the base sheet 103 and a plate 107 are in contact with each other.

Then, as illustrated in FIG. 7, the pillars 106 of the pillar array member 105 penetrate the workpiece 110, and the base sheets 101 and 103 face each other with a gap space formed by the spacers 109 therebetween. The height of the spacers 109 is set such that the workpiece 110 gets into contact with both the base sheets 101 and 103.

As illustrated in FIG. 8, the base sheet 103 is pulled up slowly (or the baes sheet 101 is pulled down slowly), so that the top and bottom (root) parts of the workpiece 110 close to the base sheets 101 and 103 are thick, and the center part thereof gets thin. The workpiece 110 is solidified in this state, and then the pillar array member 105 is pulled out. After that, the workpiece 110 is cut at the center. In the way described above, microneedles 112 are produced as illustrated in FIG. 9.

Alternatively, the base sheet 103 may be pulled up until the workpiece is torn apart at the center thereof, and then the workpiece 110 may be solidified. After that, the pillar array member 105 may be pulled out. This approach eliminates the step of cutting the workpiece at the center. The speed and distance for the pull-up may be appropriately set according to the properties of the workpiece 110 obtained after the solidification.

As described above, in the second embodiment, the workpiece in the form of particles are sandwiched between the two base sheets 101 and 103, and the pillar array member 105 is inserted in the through-holes 102 and 104 formed at approximately the center of the workpiece 110, and then the two base sheets 101 and 103 are pulled away from each other. As a result, the microneedles 112 are produced. In the manner described above, it is possible to produce the hollow microneedle sheet 110 with a simple procedure.

### (Operations and Effects)

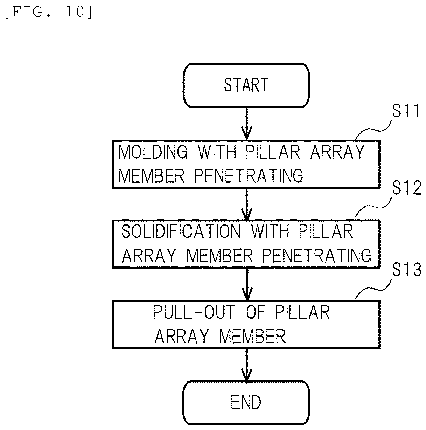

In the above configurations, as illustrated in FIG. 10, a microneedle production method according to the present invention is characterized by:
step S11 (molding step) of passing a thin pillar array member (pillar array member 5) through through-holes, and determining the shape of microneedles (microneedles 12) in the state where the pillar array member penetrates a liquid macromolecular compound (workpiece 10) in a liquid and semi-liquid state;
step S12 (solidification step) of solidifying the macromolecular compound; and
step S13 (pull-out step) of pulling out the pillar array member.

This approach makes it possible to form a hollow structure by merely pulling out the pillar array member penetrating the liquid macromolecular compound. That is to say, it is possible to produce hollow microneedles with a simple procedure.

The microneedle production method according to claim 1 is characterized in that:
a plurality of microneedles are produced simultaneously in the molding step and the solidification step; and
the pillar array member has a plate member and a plurality of pillars that respectively correspond to the plurality of microneedles and are fixed to the plate member.

This enables producing the plurality of microneedles simultaneously. That is to say, it is possible to achieve mass production with a simple procedure.

The microneedle production method is characterized in that the molding step and the solidification step further include
forming a gap between the plate member and the through-holes, and
the pull-out step further includes
moving the pillar array member in a direction closer to the through-holes and then pulling the pillar array member out of the solidified macromolecular compound.

That is, using the gap, the pillar array member is moved once in a pushing direction toward the macromolecular compound, and then the pillar array member is pulled out. This makes it easy to pull out the pillar array member.

The microneedle production method is characterized in that the molding step further includes
forming a penetrated mold by passing the thin pillar array member through the mold having recesses that define the microneedles and the through-holes formed in the recesses, such as to project from the through-holes, and then pouring the liquid macromolecular compound into the penetrated mold.

This eliminates the need of integrally forming projection portions corresponding to the pillar array member with the mold. That is, it is possible to form the penetrated mold with a simple configuration.

The microneedle production method is characterized by a molded-product release step of removing the solidified macromolecular compound as a molded product from the penetrated mold after pulling the pillar array member out of the solidified macromolecular compound.

Since the mold release is performed after the pillar array member is pulled out, it is possible to prevent breakage of the pillar array member and deformation of the molded product caused by the pillar array member even when force is applied in a direction different from the pillar array member.

The microneedle production method is characterized in that the molding step further includes
placing the liquid macromolecular compound in the form of particles on the through-holes formed in a base sheet, bringing the pillar array member, which has the pillars sticking out from the plate member, into contact with the liquid macromolecular compound with the pillar array member facing the base sheet while passing the pillar array member through the through-holes, and then pulling up the pillar array member.

This eliminates the need of the mold release from the mold. That is, it is possible to produce microneedles with a simple procedure. In addition, by selecting appropriate viscosity and thixotropy for the liquid macromolecular compound, it is possible to make the tips of the microneedles much thinner, compared with the case of using the mold. Further, the mold is not needed. That is, this approach is preferable for mass production because it needs fewer tools for the production procedure.

The microneedle production method is characterized in that the molding step further includes
placing a second base sheet that has through-holes through which the pillars pass, between the pillar array member and the liquid macromolecular compound.

This makes it possible to produce identical microneedle sheets on the upper side and on the lower side. That is, it is possible to achieve mass production of microneedles.

The microneedle production method is characterized in that the molding step further includes
pulling up the plate member until the liquid macromolecular compound is torn apart between the base sheet and the pillar array member.

This eliminates the need of cutting the liquid macromolecular compound at the center. That is, it is possible to streamline the procedure and make the tips of the microneedles much thinner.

The microneedle production method is characterized in that the solidification step further includes
solidifying the liquid macromolecular compound in the state where the liquid macromolecular compound connects the base sheet and the pillar array member, and
cutting, after the pull-out step, the solidified liquid macromolecular compound at approximately the center between the base sheet and the pillar array member

This makes it possible to adjust the diameter of the tips of the microneedles, and so to maintain the strength of the microneedles.

The microneedle production method is characterized in that the liquid macromolecular compound is in a liquid or semi-liquid state with prescribed viscosity.

Using the viscosity of the liquid macromolecular compound, it is possible to make the tips of the microneedles much longer.

The microneedle production method is characterized in that the base sheet is subjected to a surface modification treatment.

This makes it possible to adjust the wettability of the liquid macromolecular compound on the surface of the base sheet and the adhesion of the solidified microneedles to the base sheet.

The microneedle production method is characterized in that the first base sheet serves as a sheet connecting the microneedles.

This makes it possible to produce a microneedle sheet with a plurality of microneedles with a simple procedure.

### (Other Embodiments)

The above embodiments use the spacers 9, but the present invention is not limited thereto and spacers are not always needed.

### Industrial Applicability

The present invention is applicable for microneedles that are used for injections, for example.

### Reference Signs List

- 1:: Mold
- 2:: Projection-shaped recess
- 3, 102, 104:: Through-hole
- 5, 105:: Pillar array member
- 6, 106:: Pillar
- 7, 107:: Plate
- 9, 109:: Spacer
- 10, 110:: Workpiece
- 11, 111:: Microneedle sheet
- 11A:: Tip
- 11B:: Bottom surface
- 11C:: Opening
- 12, 112:: Microneedle
- 101, 103:: Base sheet

## Claims

1. A microneedle production method, comprising:
a molding step of passing a thin pillar array member through through-holes, and determining a shape of microneedles in a state where the pillar array member penetrates a liquid macromolecular compound in a liquid or semi-liquid state;
a solidification step of solidifying the macromolecular compound; and
a pull-out step of pulling the pillar array member out of the solidified macromolecular compound.

2. The microneedle production method according to claim 1, wherein:
a plurality of microneedles are produced simultaneously in the molding step and the solidification step; and
the pillar array member includes a plate member and a plurality of pillars that respectively correspond to the plurality of microneedles and are fixed to the plate member.

3. The microneedle production method according to claim 2, wherein:
the molding step and the solidification step further include forming a gap between the plate member and the through-holes; and
the pull-out step further includes moving the pillar array member in a direction closer to the through-holes and then pulling the pillar array member out of the solidified macromolecular compound.

4. The microneedle production method according to any one of claims 1 to 3, wherein the molding step further includes
forming a penetrated mold by passing the thin pillar array member through the mold such as to project from the through-holes, the mold having recesses for defining the microneedles and the through-holes respectively formed in the recesses, and
pouring the liquid macromolecular compound into the penetrated mold.

5. The microneedles production method according to any one of claims 1 to 4, further comprising
a molded-product release step of removing the solidified macromolecular compound as a molded product from the penetrated mold after pulling the pillar array member out of the solidified macromolecular compound.

6. The microneedle production method according to any one of claims 1 to 3, wherein the molding step further includes
placing the liquid macromolecular compound in a form of particles on the through-holes formed in a first base sheet, bringing the pillar array member, which has the pillars sticking out from the plate member, into contact with the liquid macromolecular compound with the pillar array member facing the first base sheet while passing the pillars of the pillar array member through the through-holes, and then pulling up the pillar array member.

7. The microneedle production method according to claim 6, wherein the molding step further includes
placing a second base sheet having through holes through which the pillars pass, between the pillar array member and the liquid macromolecular compound.

8. The microneedle production method according to claim 6 or 7, wherein the molding step further includes
pulling up the plate member until the liquid macromolecular compound is torn apart between the first base sheet and the pillar array member.

9. The microneedle production method according to claim 6 or 7, wherein the solidification step further includes
solidifying the liquid macromolecular compound in a state where the liquid macromolecular compound connects the first base sheet and the pillar array member, and
cutting the solidified liquid macromolecular compound at approximately a center between the first base sheet and the pillar array member after the pull-out step.

10. The microneedle production method according to any one of claims 6 to 9, wherein
the liquid macromolecular compound is in the liquid or semi-liquid state with prescribed viscosity.

11. The microneedle producing method according to any one of claims 6 to 10, wherein
the first base sheet is subjected to a surface modification treatment.

12. The microneedle producing method according to any one of claims 6 to 11, wherein
the first base sheet serves as a sheet connecting the microneedles.
